(19) Europäisches Patentamt
European Patent Office
Office européen des brevets

(11) Publication number: **0 235 762 A1**

(12) **EUROPEAN PATENT APPLICATION**

(21) Application number: **87102815.5**

(22) Date of filing: **27.02.87**

(51) Int. Cl.[4]: **C 07 D 215/56**, C 07 D 401/04, A 61 K 31/47, A 61 K 31/495

(30) Priority: **04.03.86 JP 45191/86**

(43) Date of publication of application: **09.09.87 Bulletin 87/37**

(84) Designated Contracting States: **BE CH DE FR GB IT LI NL SE**

(71) Applicant: **KYORIN PHARMACEUTICAL CO., LTD., No. 5, Kanda Surugadai 2-chome, Chiyoda-ku Tokyo (JP)**

(72) Inventor: **Masuzawa, Kuniyoshi, No. 5-71, Nishi-cho, Koga-shi Ibaragi-ken (JP)**
Inventor: **Suzue, Seigo, No. 13-4, Aoba 4-chome, Kuki-shi Saitama-ken (JP)**
Inventor: **Kirai, Keiji, No. 1-2-512, Aoba 1-chome, Kuki-shi Saitama-ken (JP)**
Inventor: **Ishizaki, Takayoshi, No. 9-6, Sakurada 4-chome Washimiya-machi, Kitakatsu-shika-gun Saitama-ken (JP)**

(74) Representative: **Patentanwälte TER MEER - MÜLLER - STEINMEISTER, Mauerkircherstrasse 45, D-8000 München 80 (DE)**

(54) **8-Position substituted quinolone-carboxylic acid derivatives and process for their preparation.**

(57) The present invention is concerned with certain novel quinolonecarboxylic acid derivatives of the following formula,

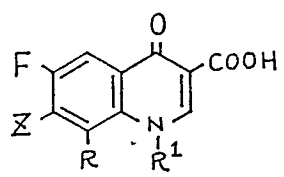

wherein R indicates chlorine, bromine, nitro, cyano, $-OR^2$, $-SR^2$ or $-NR^2R^3$ (here, $R^2$ and $R^3$ indicate hydrogen or lower alkyl each independently), $R^1$ indicates ethyl, 4-fluorophenyl or 2,4-di-fluorophenyl, Z indicates a piperazino group of the following formula,

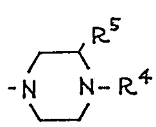

(here, $R^4$ indicates hydrogen, methyl or lower alkoxycarbonyl, $R^5$ indicates hydrogen or lower alkyl) or a pyrrolidine group of the following formula,

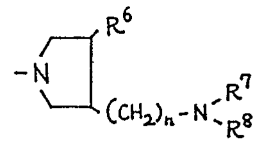

(here, n is 0 or 1, $R^6$ indicates hydrogen or methyl, $R^7$ indicates hydrogen atom or lower alkyl group, $R^8$ indicates a hydrogen, lower alkyl, lower acyl or lower alkoxycarbonyl), with the proviso that when $R^1$ is ethyl and R is chlorine, Z is not piperazino or 4-methylpiperazino; the hydrates and pharmaceutically acceptable salts thereof are useful as an antibacterial agent.

EP 0 235 762 A1

# 8-Position substituted quinolonecarboxylic acid derivatives and process for their preparation

## Detailed description of the invention:

This invention is concerned with certain novel useful quinolonecarboxylic acid derivatives represented by the formula (I),

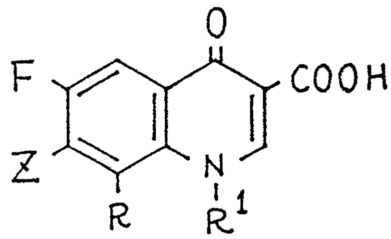

(I)

wherein R indicates chlorine, bromine, nitro, cyano, $-OR^2$, $-SR^2$ or $-NR^2R^3$ (here, $R^2$ and $R^3$ indicate hydrogen or lower alkyl each independently), $R^1$ indicates ethyl, 4-fluorophenyl or 2,4-difluorophenyl, Z indicates a piperazino group of the following formula,

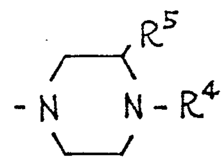

(here, $R^4$ indicates hydrogen, methyl or lower alkoxycarbonyl, $R^5$ indicates hydrogen or lower alkyl) or a pyrrolidine group of the following formula,

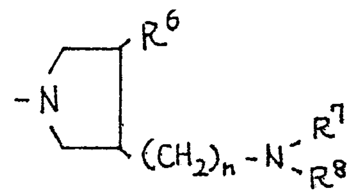

(here, n is 0 or 1, $R^6$ indicates hydrogen or methyl, $R^7$ indicates hydrogen atom or lower alkyl group, $R^8$ indicates a hydrogen, lower alkyl, lower acyl or lower alkoxycarbonyl), with the proviso that when $R^1$ is ethyl and R is chlorine, Z is not piperazino or 4-methylpiperazino; the hydrates and pharmaceutically acceptable salts thereof, with a process for their preparation, and with compositions containing them.

A lot of quinolonecarboxylic acid derivatives have ever been developed, but they mostly have hydrogen, fluorine, as a substituent at the 8 position of their quinolone ring. There is little example of quinolonecarboxylic acids with other substituents, so any influence of some substituents at the 8-position on antibacterial activity is not known.

The inventors attempted to introduce a variety of substituents into the 8-position of quinolonecarboxylic acid derivatives, and found antibacterial activity was unexpectedly expanded by it.

The compounds of the present invention have a potent antibacterial activity not only against gram-negative bacteria, but also against gram-positive bacteria and anaerobes. Therefore, they are useful antibacterial agents for the treatment of infectious human, animal or plant disease.

The compounds of the invention are synthesized through the below scheme;

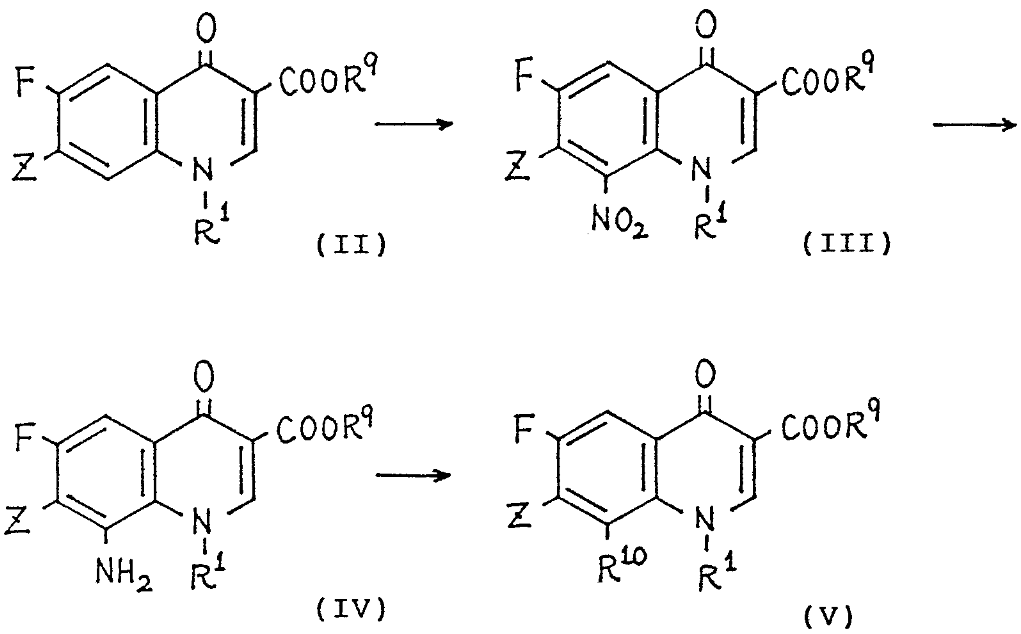

(II) (III) (IV) (V)

wherein $R^9$ indicates hydrogen or lower alkyl, $R^{10}$ indicates chlorine, bromine or cyano, $R^1$ indicates ethyl, 4-fluorophenyl or 2,4-difluorophenyl, Z indicates a piperazino group of the following formula,

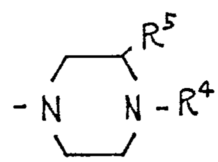

(here, $R^4$ indicates hydrogen, methyl or lower alkoxycarbonyl, $R^5$ indicates hydrogen or lower alkyl) or a pyrrolidine group of the following formula,

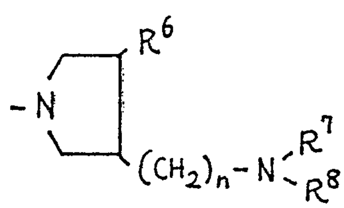

(here, n is 0 or 1, $R^6$ indicates hydrogen or methyl, $R^7$ indicates hydrogen atom or lower alkyl group, $R^8$ indicates a hydrogen, lower alkyl, lower acyl or lower alkoxycarbonyl), with the proviso that when $R^1$ is ethyl and R is chlorine, Z is not piperazino or 4-methylpiperazino.

The compound of the formula (II) is nitrated by the nitrating agent such as nitric acid and metal nitrate (an equimolar or excess amounts) in a solvent such as water, sulfuric acid, acetic acid, acetic anhydride and their mixture at -50 to 200 °C, preferably -20 to 100 °C for one or several hours to give the compound of the formula (III).

The compound of the formula (III) is hydrogenated by the reducing agent such as metal hydride, transition metal and transition metal salt, preferably by the absorption of hydrogen in the presence of the catalyst such as palladium charcoal, Raney nickel and platinum in a solvent such as water, hydrochloric acid, alcohols, acetic acid and their mixture to give the compound of the formula (IV).

Diazotization of the compound of the formula (IV) is carried out by addition of nitrous acid salt or nitrous acid ester to its aqueous solution containing an acid such as hydrochloric acid and sulfuric acid. The isolated diazonium salt or diazotizing solution is poured into the aqueous solution of metal halide or metal cyanide such as cuprous chloride, cupric chloride, cuprous bromide, cupric bromide and cuprous cyanide to give the compound of the formula (V). The halogenation is easily accomplished by treating the compound of the formula (IV) with cupric halide and

nitrous acid ester in an inert solvent such as acetonitrile and tetrahydrofuran.

The compound of the formula (I) can also be prepared through, for example, following route.

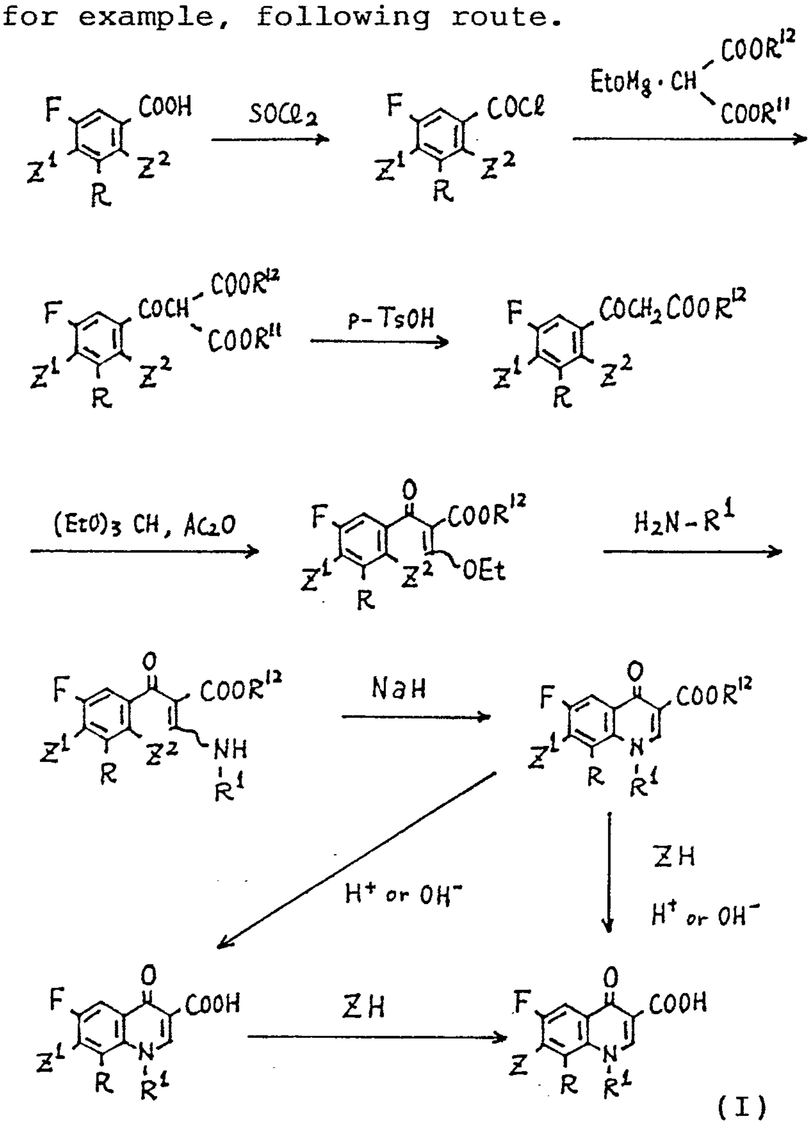


(I)

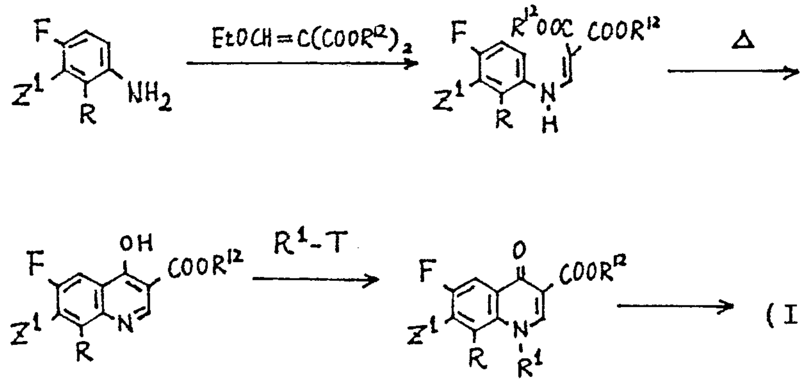


(I)

wherein $Z^2$ indicates halogen, $R^{11}$ and $R^{12}$ indicate lower alkyl each independently, T indicates leaving group, R, $R^1$, Z and $Z^1$ have the above-stated meanings.

Next, in the case of compounds in which R indicates $-OR^2$, $-SR^2$ or $-NR^2R^3$, that is, compound of the formula (VI) can be also prepared from the compounds of the formula (VII), for example, following route.

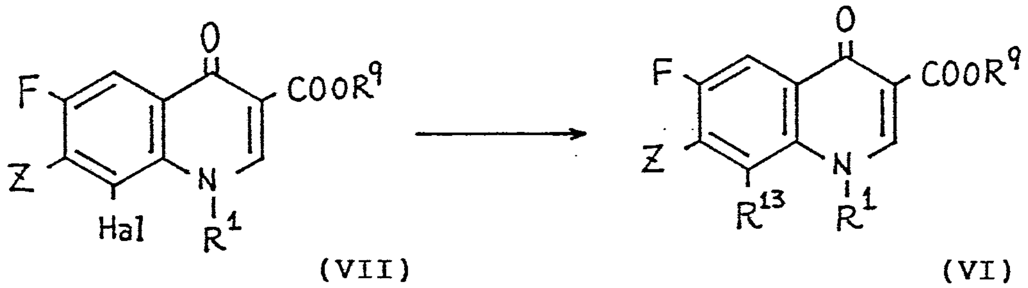

(VII) (VI)

wherein Hal indicates halogen, $R^{13}$ indicates $-OR^2$, $-SR^2$ or $-NR^2R^3$, R, $R^1$, $R^2$, $R^3$, $R^9$ and Z have the above-stated meanings.

Thus, the compound of the formula (VI) can be prepared by allowing compound of the formula (VII) to act with alkali metalic alcoholate, alkali metalic alkylthiolate, alkali metal hydroxide, alkali metal hydrogen sulfate or amine of the following formula.

$$HNR^2R^3$$

Such reaction is carried out without solvent or in the solvents such as water, alcohols, acetonitrile, dioxane, DMSO, DMF, benzene, etc. or their mixture.

In more detail, it is suitable to allow the compound of the formula (VII) to react with at least not less than equivalent moles of alkali metalic alcoholate, alkali metalic alkylthiolate, alkali metal hydroxide, alkali metal hydrogen sulfate or amine of the following formula,

$HNR^2R^3$

in 1 to 50 times volume of foregoing solvents for 1 to 100 hours at room temperature to 200 °C, and, when using low boiling point solvents, it is more advantageous to allow to react at high temperature in a sealed tube.

Moreover, in the case of compounds in which $R^9$ is lower alkyl, that is, compound of the formula (VIII),

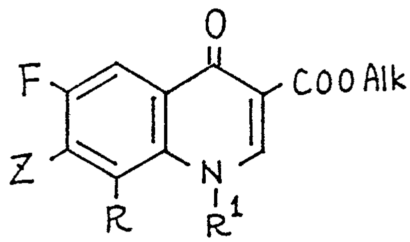

(VIII)

wherein Alk indicates lower alkyl, R, R and Z have the above-stated meanings, are converted to quinolonecarboxylic acid derivatives of the formula (I),

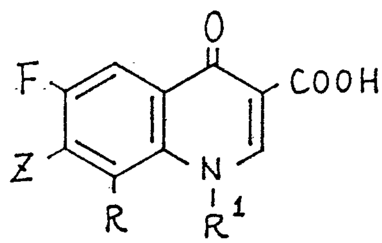

(I)

wherein R, $R^1$ and Z have the above-stated meanings, by hydrolysis according to usual method.

Such hydrolysis can be carried out easily at room temperature to boiling point of solvent in water, alcohols or mixed solutions thereof using alkalies such as sodium hydroxide and potassium hydroxide or acids such as sodium hydrochloric acid and sulfuric acid.

Furthermore, the compounds of the formula (I) can be converted, if desired, to the pharmaceutically acceptable salts by treatment with acid or alkali. The acid may be organic or inorganic acids such as, for example, hydrochloric acid, sulfuric acid, phosphoric acid, methanesulfonic acid, acetic acid, oxalic acid and lactic acid. The alkali salts may be, for example, sodium, potassium, magnesium, calcium, aluminum, cerium, chromium, cobalt, copper, iron, zinc, platinum, and silver salts.

The compound of the formula (I), the hydrates and salts thereof may be used as medicines in the conventional form of pharmaceutical preparations, which may be, for example, tablets, capsules, powder, ointments, suppositories, injections or eye drops, suitable for peroral, parenteral, enteral or local administration.

The following examples will further illustrate the invention without, however, limiting it thereto.

Reference example 1-1 Synthesis of ethyl 2-(3-chloro-2,4,5-trifluorobenzoyl)-3-(ethylamino)acrylate

A mixture of ethyl 3-chloro-2,4,5-trifluorobenzoylacrylate (2.36 g), ethyl orthoformate (2.38 g) and acetic anhydride (2.73 g) was stirred for 3 hours on an oil bath (120 to 130 °C) and then concentrated to give the brown oil (4.13 g). To a solution of this compound in ethanol (10 ml), after cooling, was added 70 % ethylamine (0.8 ml) dropwise with stirring. Stirring was continued for 1 hour at room temperature, the reacting mixture was allowed to stand at 5 °C for overnight and the resulting

precipitate was collected by filtration to give the title compound (2.36 g) as colorless needles, mp 116-117 °C.

Analysis (%) for $C_{14}H_{13}ClF_3NO_3$, Calcd. (Found): C, 50.09 (50.09); H, 3.90 (3.79); N, 4.17 (4.10).

Reference example 1-2 Synthesis of ethyl 8-chloro-1-ethyl-6,7-difluoro-1,4-dihydro-4-oxo-3-quinolinecarboxylate

A mixture of ethyl 2-(3-chloro-2,4,5-trifluorobenzoyl)-3-(ethylamino)acrylate (2.25 g) and potassium fluoride (0.58 g) in anhydrous DMF (10 ml) was stirred at 140 to 160 °C for 1 hour. After cooling, the reacting mixture was poured into ice-water (30 ml), the resulting precipitate was collected by filtration and washed with ether to give the title compound (2.21 g) as colorless needles, mp 192-193 °C.

Analysis (%) for $C_{14}H_{12}ClF_2NO_3$: Calcd. (Found): C, 53.26 (52.84); H, 3.83 (3.70); N, 4.44 (4.42).

Reference example 1-3 Synthesis of 8-chloro-1-ethyl-6,7-difluoro-1,4-dihydro-4-oxo-3-quinolinecarboxylic acid

To the mixture of acetic acid (5 ml), concentrated sulfuric acid (0.7 ml) and water (4 ml) was added ethyl 8-chloro-1-ethyl-6,7-difluoro-1,4-dihydro-4-oxo-3-quinolinecarboxylate (2.10 g) and stirred for 1 hour on an oil bath (120 °C). The reacting mixture was poured into ice-water (20 ml), the resulting precipitate was collected by filtration and washed with ether to give the title compound (1.70 g) as colorless prisms, mp 220-221 °C.

Analysis (%) for $C_{12}H_8ClF_2NO_3$, Calcd. (Found): C, 50.11

(50.15); H, 2.80 (2.72); N, 4.87 (4.81).

Reference example 2 Synthesis of ethyl 2-(3-chloro-2,4,5-trifluorobenzoyl)-3-(2,4-difluorophenylamino)acrylate

To a solution of ethyl 2-(3-chloro-2,4,5-trifluorobenzoyl)-3-ethoxyacrylate (3.08 g) in ethanol (6 ml) was added a solution of 2,4-difluoroaniline (1.27 g) in ethanol (4 m) dropwise during 15 minutes with stirring. Stirring was continued for 30 minutes below 5 °C, the resulting precipitate was collected by filtration to give the title compound (3.33 g) as white needles, mp 129-130 °C.

Analysis (%) for $C_{18}H_{11}ClF_5NO_3$, Calcd. (Found): C, 51.51 (51.72); H, 2.64 (2.52); N, 3.34 (3.36).

Reference example 3 Synthesis of ethyl 8-chloro-6,7-difluoro-1-(2,4-difluorophenyl)-1,4-dihydro-4-oxo-3-quinolinecarboxylate

A mixture of ethyl 2-(3-chloro-2,4,5-trifluorobenzoyl)-3-(2,4-difluorophenylamino)acrylate (3.13 g), sodium fluoride (0.50 g) and anhydrous DMF (12 ml) was stirred at 130 to 140 °C for 3 hours. The reacting mixture was poured into ice-water (100 ml), the resulting precipitate was collected by filtration, washed with water and recrystallized form methanol to give the title compound (2.50 g) as white powder, mp 211-213 °C.

Analysis (%) for $C_{18}H_{10}ClF_4NO_3$, Calcd. (Found): C, 54.08 (54.15); H, 2.52 (2.52); N, 3.50 (3.52).

Example 1 Synthesis of 7-(4-acetyl-1-piperazinyl)-1-ethyl-6-fluoro-1,4-dihydro-8-nitro-4-oxo-3-quinolinecarboxylic acid

To the mixture of 1-ethyl-6-fluoro-1,4-dihydro-4-oxo-7-(1-piperazinyl)-3-quinolinecarboxylic acid (20 g) in acetic acid (240 ml) was added acetic anhydride (80 ml) and stirred for 2 hours. After cooling, to the reacting mixture was added further acetic anhydride (70 ml) and then the mixture of nitric acid fuming (25 ml) and acetic anhydride (50 ml) below 0 °C. Stirring was continued for 2 hours at -5 to 0 °C and the reacting mixture was poured into ice-water. Sodium bicarbonate (55 g) was added to the mixture and extracted with chloroform. The organic layer was washed with water, dried over anhydrous sodium sulfate and concentrated under reduced pressure. The resulting residue was recrystallized from acetonitrile to give the title compound (10.91 g) as pale yellow prisms, mp 280-282 °C (decompd.).

Analysis (%) for $C_{18}H_{19}FN_4O_6$, Calcd. (Found): C, 53.20 (53.34); H, 4.71 (4.72); N, 13.79 (13.72).

Example 2 Synthesis of 1-ethyl-6-fluoro-1,4-dihydro-8-nitro-4-oxo-7-(1-piperazinyl)-3-quinolinecarboxylic acid

7-(4-Acetyl-1-piperazinyl)-1-ethyl-6-fluoro-1,4-dihydro-8-nitro-4-oxo-3-quinolinecarboxylic acid (0.2 g) was suspended in the mixture of concentrated hydrochloric acid-ethanol (1:4; 7 ml) and refluxed for 4.7 hours. The reacting mixture was concentrated under reduced pressure, neutralized with aqueous potassium carbonate solution and the resulting precipitate was collected by filtration. This precipitate was recrystallized from dimethylformamide-water to give the title compound (38 mg) as pale yellow prisms, mp 261-263.5 °C (decompd.).

Analysis (%) for $C_{16}H_{17}FN_4O_5 \cdot H_2O$, Calcd. (Found): C, 50.26 (50.09); H, 5.01 (4.83); N, 14.65 (14.41).

Example 3 Synthesis of 7-(4-acetyl-1-piperazinyl)-8-amino-1-ethyl-6-fluoro-1,4-dihydro-4-oxo-3-quinolinecarboxylic acid

7-(4-Acetyl-1-piperazinyl)-1-ethyl-6-fluoro-1,4-dihydro-8-nitro-4-oxo-3-quinolinecarboxylic acid (3 g) was suspended in the mixture of concentrated hydrochloric acid (2.5 ml)-methanol (200 ml). To this was added 10 % palladium-charcoal (0.6 g) and the mixture was agitated for 2.5 hours at room temperature in hydrogen stream (2.7 liter) under atmospheric pressure. After filtration and concentration of the reaction mixture, to the resulting residue was added ice-water (100 ml), neutralized with sodium bicarbonate and extracted with chloroform. The organic layer was washed with water, dried over anhydrous sodium sulfate and concentrated under reduced pressure. The resulting residue was recrystallized from acetonitrile to give the title compound (1.39 g) as orange prisms, mp 244-250 °C.

Analysis (%) for $C_{18}H_{21}FN_4O_4$, Calcd. (Found): C, 57.44 (57.26); H, 5.62 (5.59); N, 14.89 (14.89).

Example 4 Synthesis of 8-amino-1-ethyl-6-fluoro-1,4-dihydro-4-oxo-7-(1-piperazinyl)-3-quinolinecarboxylic acid hydrochloride

7-(4-Acetyl-1-piperazinyl)-8-amino-1-ethyl-6-fluoro-1,4-dihydro-4-oxo-3-quinolinecarboxylic acid (0.3 g) was dissolved in

6 N aqueous hydrochloric acid solution (10 ml) and heated on boiling water for 3.5 hours. The reacting mixture was concentrated, to the resulting residue was added ethanol (15 ml) and allowed to stand in a refrigerator. The resulting precipitate was collected by filtration and recrystallized from methanol to give the title compound (0.1 g) as yellow prisms, mp 283 °C (decompd.).

Analysis (%) for $C_{16}H_{19}FN_4O_3 \cdot HCl$, Calcd. (Found): C, 51.83 (51.59); H, 5.44 (5.52); N, 15.11 (14.90).

Example 5 Synthesis of 7-(4-acetyl-1-piperazinyl)-8-chloro-1-ethyl-6-fluoro-1,4-dihydro-4-oxo-3-quinolinecarboxylic acid

7-(4-Acetyl-1-piperazinyl)-8-amino-1-ethyl-6-fluoro-1,4-dihydro-4-oxo-3-quinolinecarboxylic acid (0.6 g) was dissolved in anhydrous acetonitrile (20 ml) with heating and to this was added anhydrous cupric chloride (0.27 g). To this mixture was added the solution of t-butylnitrite (0.25 g) dropwise at 60 to 65 °C during 3 minutes. After stirring for 20 minutes at same temperature, the reaction mixture was poured into chilled water (50 ml) and chloroform (200 ml) and insoluble materials were filtered off. The organic layer was washed with saturated saline, dried over anhydrous sodium sulfate and concentrated under reduced pressure. The resulting residue was recrystallized from methanol to give the title compound (90 mg) as pale green prisms, mp 265-267 °C.

Analysis (%) for $C_{18}H_{19}ClFN_3O_4 \cdot 1/3\ H_2O$, Calcd. (Found): C, 53.80 (53.78); H, 4.93 (4.77); N, 10.46 (10.51).

Example 6 Synthesis of 8-chloro-1-ethyl-6-fluoro-1,4-dihydro-4-oxo-7-(1-piperazinyl)-3-quinolinecarboxylic acid

7-(4-Acetyl-1-piperazinyl)-8-chloro-1-ethyl-6-fluoro-1,4-dihydro-4-oxo-3-quinolinecarboxylic acid (45 mg) was dissolved in 1 N aqueous sodium hydroxide solution (2.5 ml) and heated on water bath at 60 °C for 5 hours. After neutralized with acetic acid, the mixture was allowed to stand in a refrigerator. The resulting precipitate was collected by filtration and washed with water to give the title compound (20 mg) as pale yellow prisms, mp 228.5-230.5 °C (decompd.).

Mass m/e: 353 ($M^+$), 354 ($M^+$+1), 355 ($M^+$+2).

Example 7 Synthesis of 7-(4-acetyl-1-piperazinyl)-8-bromo-1-ethyl-6-fluoro-1,4-dihydro-4-oxo-3-quinolinecarboxylic acid

7-(4-Acetyl-1-piperazinyl)-8-amino-1-ethyl-6-fluoro-1,4-dihydro-4-oxo-3-quinolinecarboxylic acid (1.5 g) was dissolved in anhydrous acetonitrile (60 ml) with heating and to this was added anhydrous cupric bromide (1.5 g). To this mixture was added the solution of t-butylnitrite (0.62 g) in anhydrous acetonitrile (1 ml) dropwise at 50 to 60 °C during 10 minutes. After stirring for 5 minutes at same temperature, the reaction mixture was poured into chilled water (100 ml), extracted with chloroform. The organic layer was washed with saturated saline, dried over anhydrous sodium sulfate and concentrated under reduced pressure. The resulting residue was purified by silica gel column chromatography eluting by dichloromethane-methanol (15:1) and recrystallized from acetonitrile to give the title compound (0.34 g) as

pale yellow prisms, mp 182.5 °C (decompd.).

Analysis (%) for $C_{18}H_{19}BrFN_3O_4$, Calcd. (Found): C, 49.11 (48.87); H, 4.35 (4.27); N, 9.54 (9.69).

Example 8 Synthesis of 8-bromo-1-ethyl-6-fluoro-1,4-dihydro-4-oxo-7-(1-piperazinyl)-3-quinolinecarboxylic acid hydrochloride

7-(4-Acetyl-1-piperazinyl)-8-bromo-1-ethyl-6-fluoro-1,4-dihydro-4-oxo-3-quinolinecarboxylic acid (0.13 g) was dissolved in 1 N aqueous sodium hydroxide solution (3 ml) and heated on water bath at 60 to 80 °C for 4.5 hours. After cooling, acidified with concentrated hydrochloric acid and the mixture was allowed to stand in a refrigerator. The resulting precipitate was collected by filtration and recrystallized from dichloromethane-methanol (1:1) to give the title compound (72 mg) as colorless needles, mp 283-297 °C (decompd.).

Analysis (%) for $C_{16}H_{17}BrFN_3O_3 \cdot HCl$, Calcd. (Found): C, 44.21 (44.01); H, 4.17 (4.13); N, 9.67 (9.60).

Example 9 Synthesis of 7-(4-acetyl-1-piperazinyl)-8-cyano-1-ethyl-6-fluoro-1,4-dihydro-4-oxo-3-quinolinecarboxylic acid

To a solution of 7-(4-acetyl-1-piperazinyl)-8-amino-1-ethyl-6-fluoro-1,4-dihydro-4-oxo-3-quinolinecarboxylic acid (0.5 g) in concentrated hydrochloric acid (3 ml) was added aqueous sodium nitrite solution (0.14 g in 1 ml water) below 0 °C and stirred for 75 minutes. This solution was added dropwise to the mixture of cuprous cyanide (0.25 g), potassium cyanide (0.35 g) and sodium carbonate (2.2 g) in water (10 ml) during 15 minutes below

0 °C. Stirring was continued for 1.5 hours at this temperature, to the reacting mixture was added potassium cyanide (0.17 g) and chloroform (15 ml) and stirred for further 75 minutes at room temperature. The chloroform layer was separated, the water layer was extracted with chloroform. The organic layer was combined, washed with saturated saline and dried over sodium sulfate. After the solvent was concentrated under reduced pressure, the resulting residue was purified by silica gel column chromatography eluting by dichloromethane-methanol (10:1) and recrystallized from chloroform-methanol-concentrated aqueous ammonia (10:10:1) to give the title compound (0.1 g) as yellow needles, mp 291.5-295 °C.

Analysis (%) for $C_{19}H_{19}FN_4O_4 \cdot 1/3\ H_2O$, Calcd. (Found): C, 58.16 (58.18); H, 5.05 (4.81); N, 14.28 (14.00).

Example 10 Synthesis of 8-cyano-1-ethyl-6-fluoro-1,4-dihydro-4-oxo-7-(1-piperazinyl)-3-quinolinecarboxylic acid

7-(4-Acetyl-1-piperazinyl)-8-cyano-1-ethyl-6-fluoro-1,4-dihydro-4-oxo-3-quinolinecarboxylic acid (36 mg) was dissolved in 1 N aqueous sodium hydroxide solution (1 ml) and heated on a water bath at 60 °C for 2.5 hours. The reacting mixture was neutralized with acetic acid and allowed to stand in a refrigerator. The resulting precipitate was collected by filtration and washed with water to give the title compound (15 mg) as pale yellow prisms, mp 264.5-267 °C (decompd.).

Analysis (%) for $C_{17}H_{17}FN_4O_3 \cdot 3/2\ H_2O$, Calcd. (Found): C, 54.98 (55.10); H, 5.43 (4.99); N, 15.09 (14.65).

Mass m/e: 344 ($M^+$), 345 ($M^+$+1).

Example 11 Synthesis of 1-ethyl-6-fluoro-1,4-dihydro-8-methoxy-4-oxo-7-(1-piperazinyl)-3-quinolinecarboxylic acid

To the solution of sodium methoxide prepared from sodium (0.35 g) and absolute methanol (20 ml) was added 1-ethyl-6,8-difluoro-1,4-dihydro-4-oxo-7-(1-piperazinyl)-3-quinolinecarboxylic acid (0.96 g) and the mixture in sealed tube was stirred for 39 hours at 140 to 150 °C and then concentrated under reduced pressure. To the resulting residue was added water (14 ml), neutralized with acetic acid and the insoluble materials were filtered off. The filtrate was allowed to stand in a refrigerator, the resulting precipitate was collected by filtration and recrystallized from dichloromethane-methanol (2:1) to give the title compound (0.22 g) as pale yellow prisms, mp 163-164 °C.

Analysis (%) for $C_{17}H_{20}FN_3O_4 \cdot 6/5\ H_2O$, Calcd. (Found): C, 55.04 (55.04); H, 6.09 (5.87); N, 11.33 (11.29).

Example 12 Synthesis of 1-ethyl-6-fluoro-1,4-dihydro-8-hydroxy-4-oxo-7-(1-piperazinyl)-3-quinolinecarboxylic acid

To the solution of sodium methoxide prepared from sodium (0.8 g) and methanol (50 ml) was added 1-ethyl-6,8-difluoro-1,4-dihydro-4-oxo-7-(1-piperazinyl)-3-quinolinecarboxylic acid (3.0 g) and the mixture in sealed tube was stirred for 58 hours at 140 to 150 °C and then concentrated under reduced pressure. To the resulting residue was added water (50 ml), neutralized with acetic acid and the resulting precipitate was collected by filtration. This precipitate was purified by silica gel column chromatography eluting by chloroform-methanol-concentrated aqueous ammonia (10:10:3) and recrystallized from dichloromethane-methanol (1:5) to give the title compound (0.22 g) as pale yellow powder, mp 222 °C (decompd.).

Mass (m/e): 335 ($M^+$), 336 ($M^+$+1).

NMR ( δ in $D_2O$-NaOD): 1.31 (3H, t, J=7.0 Hz, $-CH_2C\underline{H}_2$), 3.0-3.4 (8H, m, piperazine-H), 4.96 (2H, q, J=7.0 Hz, $-C\underline{H}_2-CH_3$), 7.03 (1H, d, J=13 Hz, 5-H), 8.23 (1H, s, 2-H).

In addition, the compound of Example 11 (0.49 g) was obtained.

Example 13 Synthesis of 1-ethyl-6-fluoro-1,4-dihydro-8-methoxy-7-(4-methyl-1-piperazinyl)-4-oxo-3-quinolinecarboxylic acid

A mixture of 1-ethyl-6-fluoro-1,4-dihydro-8-methoxy-4-oxo-7-(1-piperazinyl)-3-quinolinecarboxylic acid (110 mg), sodium formate (40 mg), 87 % formic acid (0.7 ml) and 37 % formalin (45 µl) was stirred at 100 to 120 °C for 2 hours, then to the reacting mixture was added water (1 ml) and concentrated under reduced pressure. To the resulting residue was added water (0.7 ml), neutralized with 1 N aqueous sodium hydroxide solution and allowed to stand in a refrigerator. The resulting precipitate was collected by filtration and washed with water to give the title compound (51 mg) as pale blue prisms, mp 216-218.5 °C.

Analysis (%) for $C_{18}H_{22}FN_3O_4$, Calcd. (Found): C, 59.50 (59.32); H, 6.10 (6.10); N, 11.56 (11.48).

Example 14 Synthesis of 1-ethyl-6-fluoro-1,4-dihydro-8-hydroxy-4-oxo-7-(1-piperazinyl)-3-quinolinecarboxylic acid

To a solution of 1-ethyl-6-fluoro-1,4-dihydro-8-methoxy-4-oxo-7-(1-piperazinyl)-3-quinolinecarboxylic acid (300 mg) in acetic acid (4 ml) was added hydrobromic acid (4 ml) and stirred at 60 to 80 °C for 11 hours. After adding hydrobromic acid (4 ml) further, stirring was continued at same temperature for 43.5 hours. The reacting mixture was adjusted to pH 8 with concentrated aqueous ammonia, the resulting insoluble materials were filtered off and the filtrate was concentrated under reduced pressure. To the resulting residue were added small portions of water, the precipitate was collected by filtration. This precipitate was purified by silica gel chromatography eluting by chloroform-methanol-concentrated aqueous ammonia (10:10:3) and recrystallized from methanol-dichloromethane (5:1) to give the title compound (47 mg) as pale green prisms, mp 231.5-239 °C.

This compound was identical with that of Example 12 by comparison of IR spectra.

Example 15 Synthesis of 7-(3-amino-1-pyrrolidinyl)-8-chloro-1-ethyl-6-fluoro-1,4-dihydro-4-oxo-3-quinolinecarboxylic acid

A mixture of 8-chloro-1-ethyl-6,7-difluoro-1,4-dihydro-4-oxo-3-quinolinecarboxylic acid (0.5 g), 3-(t-butoxycarbonylamino)pyrrolidine (0.39 g), 1,8-diazabicyclo[5,4,0]-7-undecene (DBU, 0.29 g) and anhydrous acetonitrile (15 ml) was refluxed for 6 hours. After cooling, the resulting precipitate was collected by filtration. To the precipitate was added the solution of

concentrated hydrochloric acid-methanol (1:1, 9 ml) and stirred at room temperature for 45 minutes. The reacting mixture was neutralized with concentrated aqueous ammonia, the resulting precipitate was collected by filtration and washed with water sufficiently. The crude precipitate was recrystallized from chloroform-methanol-concentrated aqueous ammonia (40:40:3) to give the title compound (0.16 g) as colorless prisms, mp 222-224 °C (decompd.).

Analysis (%) for $C_{16}H_{17}ClFN_3O_3 \cdot 1/2\ H_2O$, Calcd. (Found): C, 52.97 (53.22); H, 5.00 (4.70); N, 11.58 (11.56).

Example 16 Synthesis of 7-(3-aminomethyl-1-pyrrolidinyl)-8-chloro-1-ethyl-6-fluoro-1,4-dihydro-4-oxo-3-quinolinecarboxylic acid

A mixture of 8-chloro-1-ethyl-6,7-difluoro-1,4-dihydro-4-oxo-3-quinolinecarboxylic acid (250 mg), 3-aminomethylpyrrolidine (105 mg) and DBU (150 mg) in anhydrous acetonitrile (4 ml) was refluxed for 2 hours. After cooling, the resulting precipitate was collected by filtration and recrystallized from chloroform-methanol-concentrated aqueous ammonia (20:6:1) to give the title compound (110 mg) as pale yellow prisms, mp 237.5-244.5 °C (decompd.).

Analysis (%) for $C_{17}H_{19}FN_3O_3 \cdot 5/3\ H_2O$, Calcd. (Found): C, 51.32 (51.11); H, 5.66 (5.28); N, 10.56 (10.40).

Example 17 Synthesis of 8-chloro-1-ethyl-6-fluoro-1,4-dihydro-7-(3-methylaminomethyl-1-pyrrolidinyl)-4-oxo-3-quinolinecarboxylic acid

A mixture of 8-chloro-1-ethyl-6,7-difluoro-1,4-dihydro-4-oxo-3-quinolinecarboxylic acid (300 mg), 3-methylaminomethylpyrrolidine (140 mg) and DBU (180 mg) in anhydrous acetonitrile (5 ml) was refluxed for 100 minutes. After cooling, the resulting precipitate was collected by filtration and recrystallized from chloroform-methanol-concentrated aqueous ammonia (10:10:3) to give the title compound (210 mg) as white powder, mp 240.5-243 °C (decompd.).

Analysis (%) for $C_{18}H_{21}FN_3O_3$, Calcd. (Found): C, 56.62 (56.61); H, 5.54 (5.51); N, 11.00 (10.88).

Example 18 Synthesis of 8-chloro-1-ethyl-7-(3-ethylaminomethyl-1-pyrrolidinyl)-6-fluoro-1,4-dihydro-4-oxo-3-quinolinecarboxylic acid

A mixture of 8-chloro-1-ethyl-6,7-difluoro-1,4-dihydro-4-oxo-3-quinolinecarboxylic acid (300 mg), 3-ethylaminomethylpyrrolidine (160 mg) and DBU (180 mg) in anhydrous acetonitrile (5 ml) was refluxed for 100 minutes. After cooling, the resulting precipitate was collected by filtration and recrystallized from dichloromethane-methanol (1:1) to give the title compound (210 mg) as white powder, mp 235-236 °C.

Analysis (%) for $C_{19}H_{23}FN_3O_3$, Calcd. (Found): C, 57.65 (57.47); H, 5.86 (5.80); N, 10.61 (10.57).

Example 19 Synthesis of 7-(cis-3-amino-4-methyl-1-pyrrolidinyl)-8-chloro-1-ethyl-6-fluoro-1,4-dihydro-4-oxo-3-quinolinecarboxylic acid

A mixture of 8-chloro-1-ethyl-6,7-difluoro-1,4-dihydro-4-oxo-3-quinolinecarboxylic acid (250 mg), cis-3-t-butoxycarbonylamino-4-methylpyrrolidine (210 mg) and DBU (150 mg) in anhydrous acetonitrile (4 ml) was refluxed for 2.5 hours. The reacting mixture was concentrated under reduced pressure. The resulting residue was dissolved in chloroform (60 ml), washed with 10 % aqueous citric acid solution and saturated saline successively, dried over sodium sulfate and concentrated under reduced pressure. The resulting residue was washed with methanol, then added to the mixture of concentrated hydrochloric acid-methanol (1:1, 6 ml) and stirred for 2 hours at room temperature. The reacting mixture was neutralized with concentrated aqueous ammonia and allowed to stand in a refrigerator. The resulting precipitate was collected by filtration, washed with chilled water sufficiently and recrystallized from dichloromethane-methanol (1:1) to give the title compound (100 mg) as white powder, mp 222-224 °C (decompd.).

Analysis (%) for $C_{17}H_{19}FN_3O_3 \cdot 1/3\ H_2O$, Calcd. (Found): C, 54.62 (54.58); H, 5.30 (5.12); N, 11.24 (11.26).

Example 20 Synthesis of 8-chloro-6,7-difluoro-1-(2,4-difluorophenyl)-1,4-dihydro-4-oxo-3-quinolinecarboxylic acid

To a mixture of concentrated sulfuric acid (1.2 ml), acetic acid (9.9 ml) in water (7.5 ml) was added ethyl 8-chloro-6,7-difluoro-1-(2,4-difluorophenyl)-1,4-dihydro-4-oxo-3-quinolinecarboxylate (2.20 g) and refluxed for 2 hours. The reacting

mixture was poured into ice-water (80 ml), the resulting precipitate was collected by filtration, washed with water sufficiently and dried to give the title compound (1.97 g) as white powder, mp 206-208 °C.

Analysis (%) for $C_{16}H_6ClF_4NO_3$, Calcd. (Found): C, 51.70 (51.66); H, 1.63 (1.52); N, 3.77 (3.81).

Example 21 Synthesis of 7-(3-t-butoxycarbonylamino-1-pyrrolidinyl)-8-chloro-6-fluoro-1-(2,4-difluorophenyl)-1,4-dihydro-4-oxo-3-quinolinecarboxylic acid

A mixture of 8-chloro-6,7-difluoro-1-(2,4-difluorophenyl)-1,4-dihydro-4-oxo-3-quinolinecarboxylic acid (200 mg), 3-t-butoxycarbonylaminopyrrolidine (150 mg), DBU (90 mg) and anhydrous acetonitrile (2 ml) was refluxed for 2 hours and then concentrated under reduced pressure. The resulting residue was dissolved in chloroform, washed with 10 % aqueous citric acid solution and saturated saline successively, dried over anhydrous sodium sulfate and concentrated under reduced pressure. The resulting residue was recrystallized from methanol to give the title compound (200 mg) as yellow powder, mp 168-170 °C.

Analysis (%) for $C_{25}H_{23}ClF_3N_3O_5$, Calcd. (Found): C, 55.82 (55.68); H, 4.31 (4.23); N, 7.81 (7.74).

Example 22 Synthesis of 7-(3-aminopyrrolidinyl)-8-chloro-6-fluoro-1-(2,4-difluorophenyl)-1,4-dihydro-4-oxo-3-quinolinecarboxylic acid

To a suspension of 7-(3-t-butoxycarbonylamino-1-pyrrolidinyl)-8-chloro-6-fluoro-1-(2,4-difluorophenyl)-1,4-dihydro-4-oxo-3-quinolinecarboxylic acid (140 mg) in methanol (2 ml) was added concentrated hydrochloric acid (2 ml) and stirred at room temperature for 30 minutes. The reacting mixture was neutralized with concentrated aqueous ammonia, the resulting precipitate was collected by filtration and washed with water, methanol and ethanol successively to give the title compound (83 mg) as white powder, mp 170-173 °C.

Analysis (%) for $C_{20}H_{15}ClF_3N_3O_3 \cdot 2\ H_2O$, Calcd. (Found): C, 50.07 (49.98); H, 4.04 (3.77); N, 8.87 (8.68).

Example 23 Synthesis of 8-chloro-6-fluoro-1-(2,4-difluorophenyl)-1,4-dihydro-4-oxo-7-(1-piperazinyl)-3-quinolinecarboxylic acid

A mixture of 8-chloro-6,7-difluoro-1-(2,4-difluorophenyl)-1,4-dihydro-4-oxo-3-quinolinecarboxylic acid (200 mg), piperazine (190 mg) and anhydrous DMSO (2 ml) was stirred at 60 to 65 °C for 30 minutes and then concentrated under reduced pressure. The resulting residue was suspended in ether, the precipitate was collected by filtration and recrystallized from dichloromethane-methanol-concentrated aqueous ammonia to give the title compound (100 mg) as yellow powder, mp 261-264 °C (decompd.).

Analysis )%) for $C_{20}H_{15}ClF_3N_3O_3 \cdot 3/4\ H_2O$, Calcd. (Found): C, 53.22 (53.23); H, 3.68 (3.38); N, 9.31 (9.21).

Example 24 Synthesis of 8-chloro-7-(3-ethylaminomethyl-1-pyrrolidinyl)-6-fluoro-1-(4-fluorophenyl)-1,4-dihydro-4-oxo-3-quinolinecarboxylic acid

A mixture of 8-chloro-6,7-difluoro-1-(4-fluorophenyl)-1,4-dihydro-4-oxo-3-quinolinecarboxylic acid (500 mg), 3-ethylaminomethylpyrrolidine (270 mg) and DBU (220 mg) in anhydrous acetonitrile (5 ml) was refluxed for 1.5 hours. After cooling, the resulting precipitate was collected by filtration, washed with methanol and ethanol successively to give the title compound (500 mg) as white powder, mp 253-255 °C (decompd.).

Analysis (%) for $C_{23}H_{22}ClF_2N_3O_3 \cdot 3/4\ H_2O$, Calcd. (Found): C, 58.11 (58.29); H, 4.98 (4.88); N, 8.84 (8.78).

Example 25 Synthesis of 8-ethoxy-1-ethyl-6-fluoro-1,4-dihydro-4-oxo-7-(1-piperazinyl)-3-quinolinecarboxylic acid

To the solution of sodium ethoxide prepared from sodium (0.32 g) and absolute ethanol (40 ml) was added 1-ethyl-6,8-difluoro-1,4-dihydro-4-oxo-7-(1-piperazinyl)-3-quinolinecarboxylic acid (1.0 g) and the mixture in sealed tube was stirred for 34.5 hours at 140 to 150 °C and then concentrated. To the resulting residue was added chilled water (10 ml), neutralized with acetic acid and then concentrated under reduced pressure. The resulting residue was purified by silica gel chromatography eluting by chloroform-methanol-concentrated aqueous ammonia (10:10:3) and recrystallized from methanol to give the title compound (0.29 g) as pale yellow prisms, mp 189.5-192 °C.

Analysis (%) for $C_{18}H_{22}FN_3O_4 \cdot 1/2\ H_2O$, Calcd. (Found): C, 58.06 (57.87); H, 6.23 (6.17); N, 11.28 (11.11).

NMR ($\delta$ in $CDCl_3$): 1.26, 1.34 (6H, t, J=7.0 Hz, $-CH_2-C\underline{H}_3$),

2.8-3.0 (4H, m, piperazine-H), 3.1-3.3 (4H, m, piperazine-H), 3.88 (2H, q, J=7.0 Hz, $-C\underline{H}_2-CH_3$), 4.55 (2H, q, J=7.0 Hz, $-C\underline{H}_2-CH_3$), 7.65 (1H, d, J=13.2 Hz, 5-H), 8.36 (1H, s, 2-H).

Example 26 Synthesis of 1-ethyl-6-fluoro-1,4-dihydro-8-methylthio-4-oxo-7-(1-piperazinyl)-3-quinolinecarboxylic acid

To a solution of 1-ethyl-6-fluoro-1,4-dihydro-4-oxo-7-(1-piperazinyl)-3-quinolinecarboxylic acid (2 g) in DMF (70 ml) was added 15 % aqueous sodium methanethiolate solution (7.8 g) and stirred at 55 to 60 °C for 5.5 hours. The reacting mixture was concentrated under reduced pressure, to the residue was added ice-water (40 ml), neutralized with acetic acid and extracted with chloroform. The organic layer was washed with water, dried over anhydrous sodium sulfate, concentrated under reduced pressure and recrystallized from methanol-dichloromethane (2:1) to give the title compound (0.43 g) as yellow prisms, mp 202.5-203.5 °C (decompd.).

Analysis (%) for $C_{17}H_{20}FN_3O_3S\cdot3/2\ H_2O$, Calcd. (Found): C, 52.03 (51.89); H, 5.91 (5.68); N, 10.71 (10.57).

Example 27 Synthesis of 1-ethyl-6-fluoro-1,4-dihydro-4-oxo-7-(1-piperazinyl)-8-(n-propoxy)-3-quinolinecarboxylic acid

To the solution of sodium n-propoxide prepared from sodium (0.4 g) and n-propanol (40 ml) was added 1-ethyl-6,8-difluoro-1,4-dihydro-4-oxo-7-(1-piperazinyl)-3-quinolinecarboxylic acid hydrate (1.0 g) and the mixture in sealed tube was stirred for 13 hours at 130 °C and then concentrated under reduced pressure. To

the resulting residue was added ice-water, neutralized with acetic acid and extracted with chloroform. The organic layer was washed with saturated saline, dried over anhydrous sodium sulfate and concentrate under reduced pressure. The resulting residue was purified by silica gel chromatography eluting by chloroform-methanol-concentrated aqueous ammonia (20:6:1) and recrystallized from dichloromethane-n-hexane to give the title compound (345 mg) as pale yellow prisms, mp 162.5-165 °C.

Analysis (%) for $C_{19}H_{24}FN_3O_4 \cdot 1/2\ H_2O$, Calcd. (Found): C, 59.06 (59.39); H, 6.52 (6.34); N, 10.87 (10.73).

Example 28 Synthesis of 8-(n-butoxy)-1-ethyl-6-fluoro-1,4-dihydro-4-oxo-7-(1-piperazinyl)-3-quinolinecarboxylic acid

To the solution of sodium n-butoxide prepared from sodium (0.3 g) and n-butanol (40 ml) was added 1-ethyl-6,8-difluoro-1,4-dihydro-4-oxo-7-(1-piperazinyl)-3-quinolinecarboxylic acid (1.0 g) and the mixture in sealed tube was stirred for 20.5 hours at 80 to 90 °C and then concentrated under reduced pressure. To the resulting residue was added ice-water (40 ml), neutralized with acetic acid and extracted with chloroform and then with dichloromethane. The organic layer was washed with saturated saline, dried over anhydrous sodium sulfate and concentrate under reduced pressure. The resulting residue was recrystallized from aqueous ethanol to give the title compound (0.51 g) as pale yellow needles, mp 190-191.5 °C.

Analysis (%) for $C_{20}H_{26}FN_3O_4$, Calcd. (Found): C, 61.37 (61.45); H, 6.69 (6.85); N, 10.73 (10.69).

Example 29 Synthesis of 8-(sec-butoxy)-1-ethyl-6-fluoro-1,4-dihydro-4-oxo-7-(1-piperazinyl)-3-quinolinecarboxylic acid

To the solution of sodium sec-butoxide prepared from sodium (0.3 g) and sec-butanol (40 ml) was added 1-ethyl-6,8-difluoro-1,4-dihydro-4-oxo-7-(1-piperazinyl)-3-quinolinecarboxylic acid (1.0 g) and the mixture in sealed tube was stirred for 11.5 hours at 70 to 100 °C and then concentrated under reduced pressure. To the resulting residue was added ice-water (50 ml), neutralized with acetic acid and extracted with chloroform and then with dichloromethane. The organic layer was washed with saturated saline, dried over anhydrous sodium sulfate and concentrate under reduced pressure. The resulting residue was recrystallized from dichloromethane-n-hexane to give the title compound (0.33 g) as white powder, mp 168-170 °C.

Analysis (%) for $C_{20}H_{26}FN_3O_4 \cdot 1/2\ H_2O$, Calcd. (Found): C, 59.99 (60.27); H, 6.80 (6.83); N, 10.49 (10.41).

Example 30 Synthesis of 8-(iso-butoxy)-1-ethyl-6-fluoro-1,4-dihydro-4-oxo-7-(1-piperazinyl)-3-quinolinecarboxylic acid

To the solution of sodium iso-butoxide prepared from sodium (0.3 g) and iso-butanol (40 ml) was added 1-ethyl-6,8-difluoro-1,4-dihydro-4-oxo-7-(1-piperazinyl)-3-quinolinecarboxylic acid (1.0 g) and the mixture in sealed tube was stirred for 6.5 hours at 80 to 100 °C and then concentrated under reduced pressure. To the resulting residue was added ice-water (40 ml), neutralized with acetic acid and extracted with chloroform and then with dichloromethane. The organic layer was washed with saturated

saline, dried over anhydrous sodium sulfate and concentrate under reduced pressure. The resulting residue was recrystallized from dichloromethane-n-hexane to give the title compound (0.58 g) as white powder, mp 187-190 °C.

Analysis (%) for $C_{20}H_{26}FN_3O_4 \cdot 1/2\ H_2O$, Calcd. (Found): C, 59.99 (60.02); H, 6.80 (6.55); N, 10.49 (10.76).

Example 31 Synthesis of 8-(tert-butoxy)-1-ethyl-6-fluoro-1,4-dihydro-4-oxo-7-(1-piperazinyl)-3-quinolinecarboxylic acid

To the solution of sodium tert-butoxide prepared from sodium (0.3 g) and tert-butanol (40 ml) was added 1-ethyl-6,8-difluoro-1,4-dihydro-4-oxo-7-(1-piperazinyl)-3-quinolinecarboxylic acid (1.0 g) and the mixture in sealed tube was stirred for 13 hours at 80 to 90 °C and then concentrated under reduced pressure. To the resulting residue was added ice-water (50 ml), neutralized with acetic acid and extracted with chloroform and then with dichloromethane. The organic layer was washed with saturated saline, dried over anhydrous sodium sulfate and concentrate under reduced pressure. The resulting residue was purified by silica gel column chromatography eluting by chloroform-methanol-concentrated aqueous ammonia (20:6:1) and recrystallized from dichloromethane-n-hexane to give the title compound (31 mg) as white yellow powder, mp 300- °C (decompd.).

Example 32 Synthesis of 8-(iso-propoxy)-1-ethyl-6-fluoro-1,4-dihydro-4-oxo-7-(1-piperazinyl)-3-quinolinecarboxylic acid

To the solution of sodium iso-propoxide prepared from sodium

(0.3 g) and iso-propanol (40 ml) was added 1-ethyl-6,8-difluoro-1,4-dihydro-4-oxo-7-(1-piperazinyl)-3-quinolinecarboxylic acid (1.0 g) and the mixture in sealed tube was stirred for 30.5 hours at 70 to 100 °C and then concentrated under reduced pressure. To the resulting residue was added ice-water (50 ml), neutralized with acetic acid and extracted with chloroform and then with dichloromethane. The organic layer was washed with saturated saline, dried over anhydrous sodium sulfate and concentrate under reduced pressure. The resulting residue was purified by silica gel column chromatography eluting by chloroform-methanol-concentrated aqueous ammonia (20:6:1) and recrystallized from dichloromethane-n-hexane to give the title compound (18 mg) as pale brown powder, mp 300- °C (decompd.).

Example 33 Synthesis of 1-ethyl-6-fluoro-1,4-dihydro-8-dimethylamino-7-(4-methyl-1-piperazinyl)-4-oxo-3-quinolinecarboxylic acid

A mixture of 7-(4-acetyl-1-piperazinyl)-8-amino-1-ethyl-6-fluoro-1,4-dihydro-4-oxo-3-quinolinecarboxylic acid (140 mg) in 1 N aqueous sodium hydroxide solution was heated up to 60 °C for 2 hours and then concentrated under reduced pressure. The resulting residue was dissolved in concentrated hydrochloric acid solution (5 ml) and then concentrated under reduced pressure again. The resulting residue was suspended in acetonitrile-methanol (1:1) and the insoluble materials were filtered off. The filtrate was concentrated under reduced pressure, the resulting residue was dissolved in hot ethanol (60 ml) and, then

cooled, the resulting precipitate was filtered off. The filtrate was concentrated under reduced pressure, to the resulting residue was added the mixture of 37 % formalin (2 ml), 87 % formic acid (2 ml) and sodium acetate (0.1 g) and stirred for 6 hours. The reacting mixture was concentrated under reduced pressure, the resulting residue was neutralized with saturated aqueous sodium bicarbonate solution, extracted with chloroform, dried over anhydrous sodium sulfate and then concentrated under reduced pressure. The resulting residue was purified by preparative thin layer chromatography and recrystallized from ethyl acetate to give the title compound (40 mg) as pale yellow prisms, mp 142-144 °C.

Analysis (%) for $C_{19}H_{25}FN_4O_3$, Calcd. (Found): C, 60.62 (60.61); H, 6.69 (6.71); N, 14.88 (14.74).

Example 34 Synthesis of 8-chloro-1-ethyl-6-fluoro-1,4-dihydro-7-(1-piperazinyl)-4-oxo-3-quinolinecarboxylic acid hydrochloride

A mixture of 8-chloro-1-ethyl-6,7-difluoro-1,4-dihydro-4-oxo-3-quinolinecarboxylic acid (0.3 g), N-methylpiperazine (0.3 g) in anhydrous DMSO (5 ml) was stirred at 60 to 70 °C for 1.5 hours and then concentrated under reduced pressure. The resulting residue was dissolved in diluted aqueous sodium hydroxide solution and then neutralized with acetic acid. The insoluble materials were filtered off, to the filtrate was added concentrated hydrochloric acid dropwise to adjust pH below 1 and allowed to stand. The resulting precipitate was collected by

filtration and washed with chilled diluted aqueous hydrochloric acid solution sufficiently to give the title compound (0.15 g) as pale yellow prisms, mp 246-254 °C.

Analysis (%) for $C_{17}H_{19}ClFN_3O_3 \cdot HCl \cdot 1/2\ H_2O$, Calcd. (Found): C, 49.41 (49.55); H, 5.12 (4.93); N, 10.17 (10.10).

Example 35 Synthesis of 8-chloro-1-ethyl-6-fluoro-1,4-dihydro-7-(3-methyl-1-piperazinyl)-4-oxo-3-quinolinecarboxylic acid hydrochloride

A mixture of 8-chloro-1-ethyl-6,7-difluoro-1,4-dihydro-4-oxo-3-quinolinecarboxylic acid (0.3 g), 2-methylpiperazine (0.3 g) in anhydrous DMSO (5 ml) was stirred at 60 to 70 °C for 1.5 hours and then concentrated under reduced pressure. The resulting residue was dissolved in diluted aqueous sodium hydroxide solution and then neutralized with acetic acid. The insoluble materials were filtered off, to the filtrate was added concentrated hydrochloric acid dropwise to adjust pH below 1 and allowed to stand. The resulting precipitate was collected by filtration and washed with chilled diluted aqueous hydrochloric acid solution sufficiently to give the title compound (0.15 g) as pale yellow prisms, mp 280-286 °C.

Analysis (%) for $C_{17}H_{19}ClFN_3O_3 \cdot HCl \cdot 1/2\ H_2O$, Calcd. (Found): C, 49.41 (49.41); H, 5.12 (4.93); N, 10.17 (10.07).

What is claimed is:

1. A compound of the formula (I);

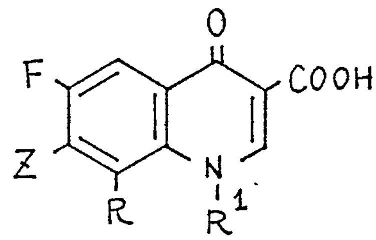

(I)

wherein R indicates chlorine, bromine, nitro, cyano, $-OR^2$, $-SR^2$ or $-NR^2R^3$ (here, $R^2$ and $R^3$ indicate hydrogen or lower alkyl each independently), $R^1$ indicates ethyl, 4-fluorophenyl or 2,4-difluorophenyl, Z indicates a piperazino group of the following formula,

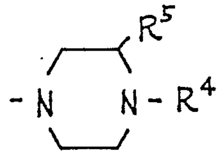

(here, $R^4$ indicates hydrogen, methyl or lower alkoxycarbonyl, $R^5$ indicates hydrogen or lower alkyl) or a pyrrolidine group of the following formula,

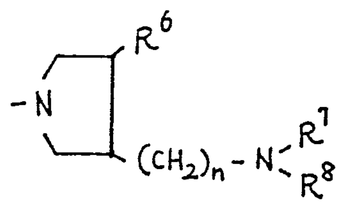

(here, n is 0 or 1, $R^6$ indicates hydrogen or methyl, $R^7$ indicates hydrogen or lower alkyl, $R^8$ indicates a hydrogen, lower alkyl, lower acyl or lower alkoxycarbonyl), with the proviso that when $R^1$ is ethyl and R is chlorine, Z is not piperazino or 4-methylpiperazino; the hydrates and pharmaceutically acceptable salts thereof.

2. A process for the preparation of a compound of the formula (III'),

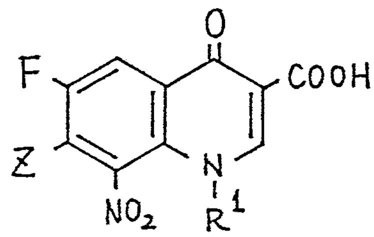

(III')

wherein $R^1$ indicates ethyl, 4-fluorophenyl or 2,4-difluorophenyl, Z indicates a piperazino group of the following formula,

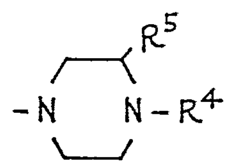

(here, $R^4$ indicates hydrogen, methyl or lower alkoxycarbonyl, $R^5$ indicates hydrogen or lower alkyl) or a pyrrolidine group of the following formula,

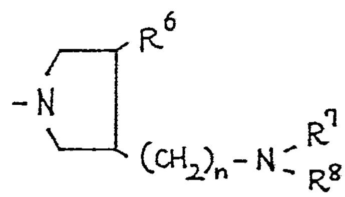

(here, n is 0 or 1, $R^6$ indicates hydrogen or methyl, $R^7$ indicates hydrogen or lower alkyl, $R^8$ indicates a hydrogen, lower alkyl, lower acyl or lower alkoxycarbonyl); which comprises nitrating the compound of the formula (II),

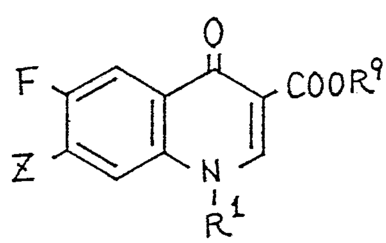

(II)

wherein $R^9$ indicates hydrogen or lower alkyl, $R^1$ and Z have the above-stated meanings; however in which, when $R^9$ is lower alkyl, the condensed product is followed by hydrolyzing to the carboxylic acid derivatives.

3. A process for the preparation of a compound of the formula (IV'),

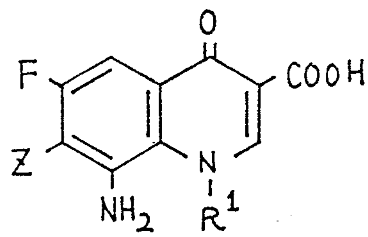

(IV')

wherein $R^1$ indicates ethyl, 4-fluorophenyl or 2,4-difluorophenyl, Z indicates a piperazino group of the following formula,

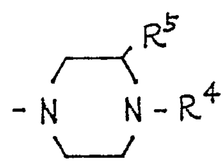

(here, $R^4$ indicates hydrogen, methyl or lower alkoxycarbonyl, $R^5$ indicates hydrogen or lower alkyl) or a pyrrolidine group of the following formula,

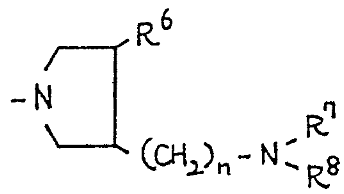

(here, n is 0 or 1, $R^6$ indicates hydrogen or methyl, $R^7$ indicates hydrogen or lower alkyl, $R^8$ indicates a hydrogen, lower alkyl, lower acyl or lower alkoxycarbonyl); which comprises reducing the compound of the formula (III),

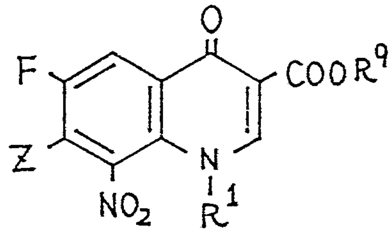

(III)

wherein $R^9$ indicates hydrogen or lower alkyl, $R^1$ and Z have the above-stated meanings; however in which, when $R^9$ is lower alkyl, the condensed product is followed by hydrolyzing to the carboxylic acid derivatives.

4. A process for the preparation of a compound of the formula (V'),

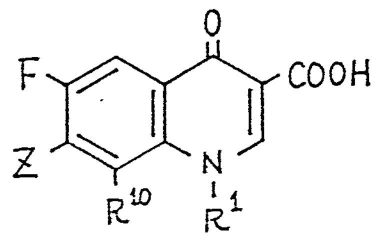

(V')

wherein $R^1$ indicates ethyl, 4-fluorophenyl or 2,4-difluorophenyl, $R^{10}$ indicates chlorine, bromine or cyano, Z indicates a piperazino group of the following formula,

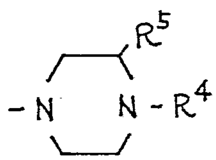

(here, $R^4$ indicates hydrogen, methyl or lower alkoxycarbonyl, $R^5$ indicates hydrogen or lower alkyl) or a pyrrolidine group of the following formula,

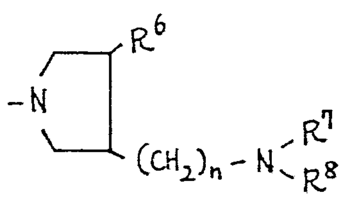

(here, n is 0 or 1, $R^6$ indicates hydrogen or methyl, $R^7$ indicates hydrogen or lower alkyl, $R^8$ indicates hydrogen, lower alkyl, lower acyl or lower alkoxycarbonyl), with the proviso that when $R^1$ is ethyl and R is chlorine, Z is not piperazino or 4-methylpiperazino; which comprises diazotizing the compound of the formula (IV),

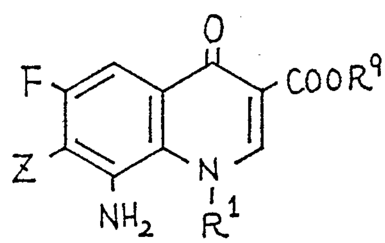

(IV)

wherein $R^9$ indicates hydrogen or lower alkyl, $R^1$ and Z have the above-stated meanings, and followed to reacting resulting product with metal halogenate or metal cyanide; however in which, when $R^9$ is lower alkyl, the condensed product is followed by hydrolysis to the carboxylic acid derivatives.

5. A process for the preparation of a compound of the formula (VII),

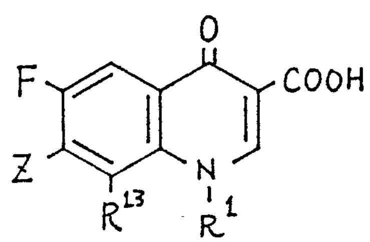

(VII)

wherein $R^1$ indicates ethyl, 4-fluorophenyl or 2,4-difluorophenyl, $R^{13}$ indicates $-OR^2$, $-SR^2$ or $-NR^2R^3$ (here, $R^2$ and $R^3$ indicate hydrogen or lower alkyl each independently), Z indicates a piperazino group of the following formula,

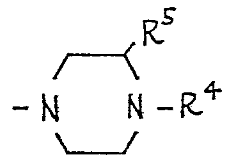

(here, $R^4$ indicates hydrogen, methyl or lower alkoxycarbonyl, $R^5$ indicates hydrogen or lower alkyl) or a pyrrolidine group of the following formula,

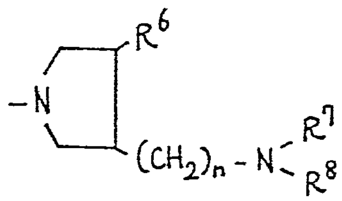

(here, n is 0 or 1, $R^6$ indicates hydrogen or methyl, $R^7$ indicates hydrogen, lower alkyl, $R^8$ indicates hydrogen, lower alkyl, lower acyl or lower alkoxycarbonyl); which comprises a compound of the formula (VI);

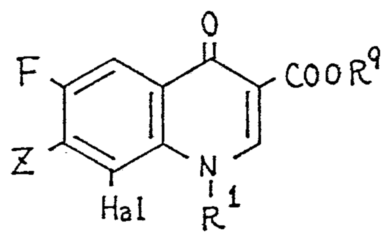

(VI)

wherein Hal indicates halogen, $R^9$ indicates hydrogen or lower alkyl, $R^1$ and Z have the above-stated meanings, with alkali metalic alcoholate, alkali metalic alkylthiolate, alkali metal hydroxide, alkali metal hydrogen sulfate or amine of the following formula,

$$HNR^2R^3$$

(here, $R^2$ and $R^3$ have the above-stated meanings); however, in which, when $R^9$ is lower alkyl, the condensed product is followed by hydrolysis to the carboxylic acid derivatives.

6. A process for the preparation of a compound of the formula (I),

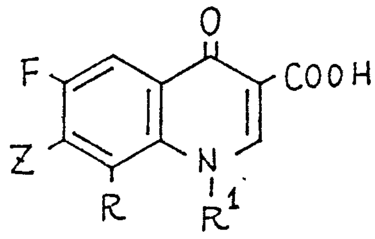

(I)

wherein R indicates chlorine, bromine, nitro, cyano, $-OR^2$, $-SR^2$ or $-NR^2R^3$ (here, $R^2$ and $R^3$ indicate hydrogen or lower alkyl each independently), $R^1$ indicates ethyl, 4-fluorophenyl or 2,4-difluorophenyl, Z indicates a piperazino group of the following formula,

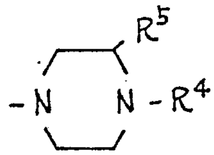

(here, $R^4$ indicates hydrogen, methyl or lower alkoxycarbonyl, $R^5$ indicates hydrogen or lower alkyl) or a pyrrolidine group of the following formula,

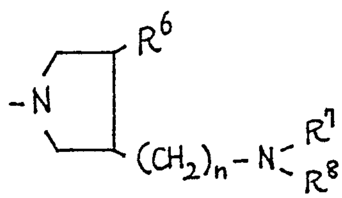

(here, n is 0 or 1, $R^6$ indicates hydrogen or methyl, $R^7$ indicates hydrogen or lower alkyl, $R^8$ indicates a hydrogen, lower alkyl, lower acyl or lower alkoxycarbonyl), with the proviso that when $R^1$ is ethyl and R is chlorine, Z is not piperazino or 4-methylpiperazino; which comprises a compound of the formula (IX);

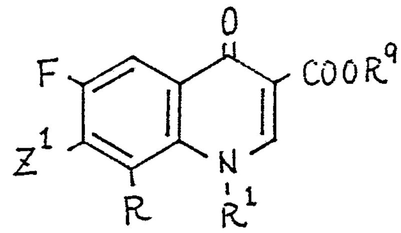

(IX)

wherein $R^9$ indicates hydrogen or lower alkyl, $Z^1$ indicates a halogen atom, R and $R^1$ have the above-stated meanings; with a compound of the formula (VII);

Z-H

wherein Z have the above-stated meanings; however in which, when $R^9$ is lower alkyl, the condensed product is followed by hydrolysis to the carboxylic acid derivatives.

7. An antibacterial pharmaceutical composition comprising at least one compound according to claim 1 and an inert pharmaceutical acceptable carrier.

European Patent Office

**EUROPEAN SEARCH REPORT**

0 235 762

EP 87102815.5

**DOCUMENTS CONSIDERED TO BE RELEVANT**

| Category | Citation of document with indication, where appropriate, of relevant passages | Relevant to claim | CLASSIFICATION OF THE APPLICATION (Int. Cl 4) |
|---|---|---|---|
| A | EP - A2 - 0 106 489 (WARNER-LAMBERT)<br>* Abstract; claims 1,9,12,18 *<br>-- | 1,6,7 | C 07 D 215/56<br>C 07 D 401/04<br>A 61 K 31/47<br>A 61 K 31/495 |
| A | GB - A - 2 057 440 (KYORIN)<br>* Claims 1,9,15 *<br>-- | 1,6,7 | |
| A | US - A - 4 528 287 (YASUO ITOH)<br>* Claims 1,9 *<br>---- | 1,7 | |
| | | | **TECHNICAL FIELDS SEARCHED (Int. Cl 4)** |
| | | | C 07 D 215/00<br>C 07 D 401/00 |

The present search report has been drawn up for all claims

| Place of search | Date of completion of the search | Examiner |
|---|---|---|
| VIENNA | 09-06-1987 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82